(19) Europäisches Patentamt
European Patent Office
Office européen des brevets

(11) Veröffentlichungsnummer: **0 144 815**
**B1**

# (12) EUROPÄISCHE PATENTSCHRIFT

(45) Veröffentlichungstag der Patentschrift: **24.09.86**

(51) Int. Cl.⁴: **C 07 C 43/303,** C 07 C 41/56, A 61 K 7/46, C 11 B 9/00

(21) Anmeldenummer: **84113663.3**

(22) Anmeldetag: **13.11.84**

**ERRATUM**

| DIE TEXTSTELLE: / TEXT PUBLISHED: / LE PASSAGE SUIVANT: | (SEITE, SPALTE, ZEILE) (PAGE, COLUMN, LINE) (PAGE, COLONNE, LIGNE) | | | LAUTET BERICHTIGT: / SHOULD READ: / DEVRAIT ETRE LU: |
|---|---|---|---|---|
| Dominierend ist jedoch eine leicht bittere Zitruskomponente, die an Grape- Wirkstoff verleiht fruchtigen und blumigen Kompositionen Frische und Süße. | 3 | 3 | 17-20 | Dominierend ist jedoch eine lei bittere Zitruskomponente, die a Grapefruitschale und Bergamottč erinnert. Der erfindungsgemäße Wirkstoff verleiht fruchtigen u blumigen Kompositionen Frische und Süße. |

Tag der Entscheidung über die Berichtigung / Date of decision on rectification: / Date de décision portant sur modification: 04.11.86

Ausgabe- und Veröffentlichungstag: / Issue and publication date: / Date d'edition et de publication: 07.01.87

Patbl.Nr / EPB no: / Bull. no: 87/02

(19) Europäisches Patentamt
European Patent Office
Office européen des brevets

(11) Veröffentlichungsnummer: **0 144 815**
**B1**

(12) # EUROPÄISCHE PATENTSCHRIFT

(45) Veröffentlichungstag der Patentschrift: **24.09.86**

(51) Int. Cl.⁴: **C 07 C 43/303**, C 07 C 41/56, A 61 K 7/46, C 11 B 9/00

(21) Anmeldenummer: **84113663.3**

(22) Anmeldetag: **13.11.84**

(54) **Alpha-tertiäre Dimethylacetale, ihre Herstellung und Verwendung als Riechstoffe.**

(30) Priorität: **17.11.83 DE 3341605**

(43) Veröffentlichungstag der Anmeldung: **19.06.85 Patentblatt 85/25**

(45) Bekanntmachung des Hinweises auf die Patenterteilung: **24.09.86 Patentblatt 86/39**

(84) Benannte Vertragsstaaten: **CH DE FR GB LI NL**

(56) Entgegenhaltungen:
**EP-A-0 076 493**
**US-A-3 493 621**

**CHEMICAL ABSTRACTS, Band 53, Nr. 11, 10. Juni 1959, Spalte 10273g, Columbus, Ohio, US; L. RE u.a.: "Cyclization of 3-carboxy-3,6-dimethyl-1,5-heptadiene, a terpene acid with the skeleton of artemisia ketone"**

(73) Patentinhaber: **Consortium für elektrochemische Industrie GmbH, Zielstattstrasse 20, D-8000 München 70 (DE)**

(72) Erfinder: **Gebauer, Helmut, Dr., Schaffhauser Strasse 18, D-8000 München 71 (DE)**
Erfinder: **Regiert, Marlies, Schraudolphstrasse 2a, D-8000 München 40 (DE)**

**EP 0 144 815 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft eine Auswahl von α-tertiären Dimethylacetalen, deren Herstellung und Verwendung als Riechstoffe.

2.5-Dimethyl-2-vinyl-4-hexenal ist gemäß CA 53 (1959) 10274 f bekannt. Die Verbindung riecht nach Zitrone. Ferner ist aus CA 53 10274 g das entsprechende Diethylacetal bekannt, das einen schwachen Geruch nach grünen Blättern aufweist.

Es sind weiterhin Dampfdruckmessungen veröffentlicht worden, die unter anderem an den Riechstoff 2.2.5-Trimethyl-4-hexenal durchgeführt wurden. Hierzu sei auf CA 82 (1975) 64321 f verwiesen.

Ferner ist gemäß DE-OS 31 39 358 2.2-Dimethyl-3-phenyl-propanal als Duftstoff bekannt.

Die vorstehend aufgeführten Aldehyde sind nicht alkalistabil und somit für die Parfümierung einer Vielzahl von Produkten z.B. Seifen oder Waschmitteln - nicht brauchbar.

Aufgabe der Erfindung war es, alkalistabile Duftstoffe aufzufinden, die unter anderem zur Parfümierung von Seifen, Waschmitteln, sowie als Bestandteil alkalistabiler Colognes eingesetzt werden können und auch eine für diesen Verwendungszweck geeignete Dufttönung aufweisen.

Es wurde nun mit einer engen Auswahl von-α-tertiären Dimethylacetalen Duftstoffe aufgefunden, die der genannten Aufgabenstellung gerecht werden.

Gegenstand der Erfindung sind 1.1-Dimethoxy-2.5-dimethyl-2-vinyl-4-hexen, 1.1-Dimethoxy-2.2.5-trimethyl-4-hexen und 1.1-Dimethoxy-2.2-dimethyl-3-phenyl-propan.

Die erfindungsgemäßen Verbindungen lassen sich durch folgende allgemeine Formel darstellen

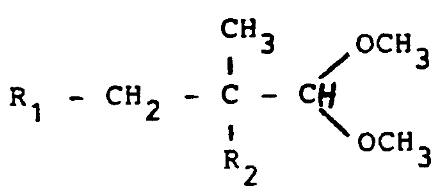

wobei $R_1$ die 2.2-Dimethylvinyl - Gruppe ist, wenn $R_2$ für Methyl oder Vinyl steht und ferner $R_1$ die Phenylgruppe ist, wenn $R_2$ Methyl bedeutet.

Die erfindungsgemäßen Verbindungen sind durch Acetalisierung der entsprechenden Aldehyde mit Methanol zugängig. Ein bevorzugtes Verfahren zur Herstellung der erfindungsgemäßen Verbindungen ist dadurch gekennzeichnet, daß 2.5-Dimethyl-2-vinyl-4-hexenal, 2.2.5-Trimethyl-4-hexenal oder 2.2-Dimethyl-3-phenyl-propanal mit Methanol in Gegenwart von wasserfreien Calciumsalzen umgesetzt werden.

Methanol wird zumindest in äquimolaren Mengen, also in Mengen von mindestens 2 Mol pro 1 Mol umzusetzenden Acetals eingesetzt. Zumeist dient jedoch Methanol auch als Lösungsmittel und liegt somit in höherem Überschuß vor.

Beispiele für wasserfreie Calziumsalze sind Calziumchlorid, Calziumcarbonat, Calziumoxid, Calziumhydroxid, Calziumphosphat, insbesondere Calziumchlorid und Calziumoxid. Die Menge an einzusetzendem Calziumsalz wird so bemessen, daß sie zumindest die an sich bei der Reaktion freiwerdende Menge an Wasser zu binden vermag. Sie beträgt pro Mol umzusetzenden Aldehyds 0,1 bis 1 Mol, insbesondere 0,2 bis 0,4 Mol.

Zur Reaktionsbeschleunigung werden dem Reaktionsgemisch oftmals noch katalytisch wirksame Mengen an Ammoniumnitrat zugesetzt. Es sind dies insbesondere 0,01 bis 0,1 Mol pro molaren Ansatz an Aldehyd.

Die als Ausgangsverbindungen einzusetzenden Aldehyde sind im Eintopfverfahren aus Basischemikalien zugänglich: 2.5-Dimethyl-2-vinyl-4-hexenal ist durch Umsetzen von Tiglinaldehyd mit Prenylchlorid 2.2.5-Trimethyl-4-hexenal durch Umsetzen von Isobutyraldehyd mit Prenylchlorid und 2.2-Dimethyl-3-phenyl-propanal durch Umsetzen von Isobutyraldehyd mit Benzylchlorid in einem organische/alkalischen 2-Phasensystem in Gcgenwart eines Phasentransfer-Katalysators zugänglich.

Es werden pro 1 Mol Isobutyraldehyd bzw. Tiglinaldehyd etwa 1 bis 1,2 Mol Prenylchlorid bzw. Benzylchlorid eingesetzt.

Das organisch/alkalische 2-Phasensystem wird gebildet aus einem organischen, mit Wasser nicht mischbaren, inerten Lösungsmittel und einer 5 bis 50 %-igen, wäßrigen Lösung oder in fester Form vorliegenden Alkalimetallhydroxids.

Als Phasentransferkatalysatoren werden beispielsweise Kronenether, quartäre Ammonium- und Phosphoniumsalze eingesetzt in Mengen von 0,5 bis 5 Molprozent, bezogen auf Prenylchlorid bzw. Benzylchlorid.

Die Reaktionstemperaturen betragen 20° bis 150° C, insbesondere 60° bis 70° C.

Zweckmäßigerweise wird das Verfahren so durchgeführt, daß das 2-Phasensystem mit dem Katalysator vorgelegt und ein Gemisch der Reaktionskomponenten zugetropft wird.

Der vorstehend beschriebene Syntheseweg stellt das besonders bevorzugte Verfahren zur Herstellung der erfindungsgemäßen Dimethylacetale dar, das dadurch gekennzeichnet ist, daß

a) Isobutyraldehyd bzw. Tiglinaldehyd und Prenylchlorid bzw. Benzylchlorid im Eintopfverfahren in einem organisch/alkalischen 2-Phasensystem in Gegenwart eines Phasentransferkatalysators umgesetzt werden und

b) der gemäß a) erhaltene Aldehyd mit Methanol in Gegenwart von wasserfreien Calziumsalzen umgesetzt wird.

1.1-Dimethoxy-2.5-dimethyl-2-vinyl-4-hexen ist ein Riechstoff von süßem, fruchtigem, leicht bitterem Angeruch, der eine ausgeprägte Spitze von Zitrone aufweist. Bestimmend ist die

natürlich wirkende Duftcharakteristik mit Fond-Noten von Bergamottöl, Petitgrainöl, Orangenöl bitter sowie blumigen Akzenten. Der erfindungsgemäße Duftstoff harmoniert mit Duftkomplexen, wie Neroli, Bergamott, Lavendel und Zitrone. Sein bevorzugtes Einsatzgebiet sind Seifen- und alkalibeständige Colognes sowie fruchtig- blumige Kompositionen.

1.1-Dimethoxy-2.2.5-trimethyl-4-hexen ist ein Duftstoff von frisch-fruchtigem, an Grapefruit vor allem erinnernden Geruch. Der erfindungsgemäße Duftstoff gibt Kompositionen wie Fougère, Chypre, Eau de Cologne frische, lebhafte Kopfnoten.

1.1-Dimethoxy-2.2-dimethyl-3-phenyl-propan ist ein Duftstoff von süß-fruchtigem Geruch mit blumigen Nuancen. Dominierend ist jedoch eine leicht bittere Zitruskomponente, die an Grape-Wirkstoff verleiht fruchtigen und blumigen Kompositionen Frische und Süße. Aufgrund seiner guten Haftung ist er besonders als Herz- und Basisnote wertvoll.

Die erfindungsgemäßen Riechstoffe werden zur Parfümierung kosmetischer und technischer Produkte allein und insbesondere in Kombination mit anderen Riechstoffen eingesetzt. Die Duftstoffe sind alkalistabil. Es sind somit Anwendungsgebiete eröffnet, unter anderen auf dem Sektor der Waschmittel, der Seifen, der Haarkosmetik und der seifenstabilen Colognes.

Die Erfindung wird nun anhand von Beispielen näher erläutert:

Soweit sich im folgenden Zahlenangaben auf die Zusammensetzung von Duftstoffmischungen beziehen, sollen darunter Gewichtsteile verstanden werden.

**Beispiel 1**

Herstellung von 1.1-Dimethoxy-2.2-dimethyl-3-phenyl-propan. In eine Vorlage aus 162 g (1 Mol) 2.2-Dimethyl-3-phenyl-propanal, 5 g Ammoniumnitrat und 300 ml Methanol wurden portionsweise 37 g wasserfreies Calziumchlorid unter Rühren zugegeben. Danach wurde noch mit 1 ml 85 %-iger Phosphorsäure versetzt. Die Reaktionstemperatur betrug 40° C. Nach 6 Stunden war die Reaktion beendet. Schließlich wurde das Reaktionsgemisch mit 2 n-Natronlauge neutral gestellt und die anorganische phase abgetrennt. Zur Aufarbeitung der organischen Phase wurde zunächst Methanol abgezogen und anschließend über eine Vigreux-Kolonne abdestilliert. Das Zielprodukt wurde als farblose Flüssigkeit bei 120° C und 16 mbar Druck erhalten. Die Ausbeute betrug 156 g entsprechend 75 % der Theorie. Brechungsindex bei 20° C 1,494.

**Beispiel 2**

Herstellung von 1.1-Dimethoxy-2.5-dimethyl-2-vinyl-4-hexen. Es wurde die Arbeitsweise gemäß Beispiel 1 wiederholt, mit der Abänderung, daß anstatt 1 Mol 2.2-Dimethyl-3-phenyl-propanal 1 Mol 2.5-Dimethyl-2-vinyl-4-hexénal eingesetzt wurden.

Das Zielprodukt wurde als farblose Flüssigkeit bei 94° C und 16 mbar erhalten. Die Ausbeute betrug 178,5 g entsprechend 90 % der Theorie.

Brechungsindex bei 20° C 1,456.

**Beispiel 3**

Herstellung von 1.1-Dimethoxy-2.2.5-trimethyl-4-hexen. Es wurde die Arbeitsweise gemäß Beispiel 1 wiederholt, mit der Abänderung, daß anstatt 2.2-Dimethyl-3-phenyl-propanal 1 Mol 2.2.5-Trimethyl-4-hexenal eingesetzt wurden.

Das Zielprodukt wurde bei 82° C und 16 mbar als farblose Flüssigkeit erhalten. Die Ausbeute betrug 149 g entsprechend 80 % der Theorie.

Brechungsindex bei 20° C 1,441.

**Beispiel 4**

1.1-Dimethoxy-2.2-Dimethyl-3-phenyl-propan als Riechstoff. Süß-blumiges Parfümöl

| | a | b |
|---|---|---|
| Phenylethylalkohol | 70 | 70 |
| Hydroxycitronellal | 200 | 200 |
| Verdyl acetat (Givaudan Corp.) | 60 | 60 |
| Sandelholzöl | 55 | 55 |
| Jasmin synth. | 80 | 80 |
| Keton-Moschus | 40 | 40 |
| Citronellol | 60 | 60 |
| Ylang-ylang-öl | 35 | 35 |
| Methylionon | 50 | 50 |
| Benzylpropionat | 60 | 60 |
| Geraniol | 40 | 40 |
| 1.1-Dimethoxy-2.2-dimethyl-3-phenyl-propan | - | 250 |
| | 750 | 1000 |

Die blumige Komposition gemäß a gewinnt durch Zugabe von 250 Gew.-Tl. 1.1-Dimethoxy-2.2-dimethyl-3-phenyl-propan (gemäß b) an Strahlkraft und Natürlichkeit. Die Ionon-Note wird unterstrichen, die fruchtigen Nuancen verstärkt.

**Beispiel 5**

1.1-Dimethoxy-2.2-dimethyl-3-phenyl-propan als Riechstoff.
Parfümöl mit Fougère-Note

| | a | b |
|---|---|---|
| Bergamottöl | 200 | 200 |
| Coumarin | 220 | 220 |
| Lavandinöl | 100 | 100 |
| Vetiveröl | 20 | 20 |
| Linalylacetat | 30 | 30 |
| Terpinylacetat | 30 | 30 |
| Phenylethylalkohol | 50 | 50 |
| Geraniumöl | 30 | 30 |
| Eichenmoss-Extrakt | 10 | 10 |
| Patschouliöl | 20 | 20 |
| Galaxolide (I.F.F.) | 20 | 20 |
| Benzylacetat | 40 | 40 |
| α-Amyl-zimtaldehyd | 30 | 30 |
| 1.1.-Dimethoxy-2.2-dimethyl-3-phenyl-propan | - | 200 |
| | 800 | 1000 |

Der Duftkomplex gemäß a ist ein Fougère-Typ mit vorherrschender Coumarin-Note. Duch Zusatz von 200Gew.-Tl. 1.1-Dimethoxy-2.2-dimethyl-3-phenyl-propan (gemäß b) wird der warm-würzige Charakter zurückgedrängt, die Komposition wirkt strahlender. Eine pudrige Süße erhöht den Geruchseffekt der Base.

**Beispiel 6**

1.1-Dimethoxy-2.5-dimethyl-2-vinyl-4-hexen als Riechstoff.
Parfümöl mit frischer Blumennote

| | a | b |
|---|---|---|
| Hydroxycitronellal | 150 | 150 |
| Bergamottöl | 200 | 200 |
| Jasmin synth. | 120 | 120 |
| 6-Methyl-alpha-Ionon | 120 | 120 |
| Benzylacetat | 100 | 100 |
| Heliotropin | 70 | 70 |
| Dimethylbenzylcarbinol | 30 | 30 |
| Fenchelöl (süß) | 20 | 20 |
| Eichenmoss-Extrakt | 20 | 20 |
| Benzylbenzoat | 60 | 60 |
| 1.1-Dimethoxy-2.5-dimethyl-2-vinyl-4-hexen | - | 110 |
| | 890 | 1000 |

Das Parfümöl gemäß a kann als süß-blumig beschrieben werden und erinnert an Maiglöckchenduft. Durch Zugabe von 110 Gew.-Tl. 1.1-Dimethoxy-2.5-dimethyl-2-vinyl-4-hexan (gemäß b) erhält das süß-blumige Geruchsbild eine lebendige, frische und natürliche Kopfnote.

**Beispiel 7**

1.1-Dimethoxy-2.5-dimethyl-2-vinyl;4-hexen als Riechstoff. Parfümöl mit citronig frischer Note

| | a | b |
|---|---|---|
| Litsea Cubebaöl chin. | 170 | 170 |
| Grapefruitöl | 180 | 180 |
| Citronellol | 160 | 160 |
| Bergamottöl | 60 | 60 |
| Citronenöl | 100 | 100 |
| Sandalwoodöl | 20 | 20 |
| Ethylvanillin | 16 | 16 |
| Coumarin | 10 | 10 |
| Heliotropin | 20 | 20 |
| Methylanthranilat | 34 | 34 |
| iso-Eugenol | 40 | 40 |
| Geraniol | 90 | 90 |
| 1.1-Dimethoxy-2.5-dimethyl-2-vinyl-4-hexen | - | 100 |
| | 900 | 1000 |

Das Parfümöl gemäß a ist vom Agrumen-Typ und mit blumigen Tönen unterlegt. Der durch Zusatz von 100 Gew.-Tl. 1.1-Dimethoxy-2.5-dimethyl-2-vinyl-4-hexen erhaltene Duftkomplex b wirkt im Angeruch angenehm frisch-herb. Neben den blumigen Akzenten kommt verstärkt die fruchtige Note zum Tragen.

**Beispiel 8**

1.1-Dimethoxy-2.2.5-trimethyl-4-hexen als Riechstoff. Parfümöl mit Fougère-Note

| | a | b |
|---|---|---|
| Bergamottöl | 230 | 230 |
| Coumarin | 230 | 230 |
| Spiköl spanisch | 110 | 110 |
| Terpinylacetat | 30 | 30 |
| Linalylacetat | 30 | 30 |
| Baummoos abs. 5 % in DPG | 60 | 60 |
| Patschouliöl | 20 | 20 |
| Keton-Moschus | 40 | 40 |
| Phenylethylalkohol | 60 | 60 |
| Vetiveröl, Java | 30 | 30 |
| Benzylacetat | 40 | 40 |
| alpha-Amyl-zimtaldehyd | 30 | 30 |
| 1.1-Dimethoxy-2.2.5-trimethyl-4-hexen | - | 90 |
| | 910 | 1000 |

Das Parfümöl gemäß a besitzt einen süßen, würzigen Grundgeruch mit holziger Nebennote. Die durch Zugabe von 90 Gew.-Tl. 1.1-Dimethoxy-2.2.5-trimethyl-4-hexen erhaltene Base b wirkt gegenüber a kräftiger und frischer. Sie eignet sich als Herren-Cologne.

**Beispiel 9**

1.1-Dimethoxy-2.2.5-trimethyl-4-hexen als Riechstoff. Parfümöl Richtung "Chypre"

| | a | b |
|---|---|---|
| Hydroxycitronellal | 110 | 110 |
| Bergamottöl | 130 | 130 |
| cis Jasmon | 90 | 90 |
| Phenylethylalkohol | 90 | 90 |
| alpha-Hexylzimtaldehyd | 70 | 70 |
| Methylionon | 50 | 50 |
| Ambrettemoschus | 60 | 60 |
| Eichenmoos-Extrakt | 40 | 40 |
| Styrallylacetat | 15 | 15 |
| gamma-Decalacton | 10 | 10 |
| Civette 5 % in DPG | 40 | 40 |
| Linalool | 55 | 55 |
| Sandelholz | 40 | 40 |
| Orangenöl, bitter | 40 | 40 |
| Ethylvanillin | 30 | 30 |
| Benzylacetat | 40 | 40 |
| 1.1-Dimethoxy-2.2.5-trimethyl-4-hexen | - | 90 |
| | 910 | 1000 |

Der feminine Duftkomplex gemäß a läßt sich als weich, süß und leicht blumig charakterisieren. Durch Zugabe von 90 Gew.-Tl. 1.1-Dimethoxy-2.2.5-trimethyl-4-hexen gewinnt der Duftkomplex eine lebhafte Kopfnote. Die Geruchscharakteristik ist blumig und frisch.

**Beispiel 10**

Stabilitätstest

1.1-Dimethoxy-2.5-dimethyl-2-vinyl-4-hexen, 1.1-Dimethoxy-2.2.5-trimethyl-4-hexen und 1.1-Dimethoxy-2.2-dimethyl-3-phenyl-pro-pan wurden in neutralen und alkalischen Medien 60 Tage bei 40° C aufbewahrt. Danach wurde auf Farb- und Geruchsveränderungen geprüft und mit frisch bereiteten, unbehandelten Lösungen verglichen. Die Lösungen wurden ferner mittels Dünnschichtchromatographie untersucht.

Es wurden bei jeweils 3 Wiederholungen 0,5 Gew.-%-ige Lösungen der erfindungsgemäßen Riechstoffe in 60 Gew.-%-igem wäßrigem Ethanol bereitet. Die alkalischen Lösungen wurden mit 0,1 n NaOH auf einen pH-Wert von 14 gebracht.

Testlösung 1 pH = 7

Testlösung 2 pH = 14

Unter den oben beschriebenen Bedingungen waren die drei erfindungsgemäßen Riechstoffe in allen Testlösungen stabil: Es konnten keine Farb- oder Geruchsveränderungen festgestellt werden. Die Dünnschichtchromatogramme ergaben keinen Hinweis auf chemische Veränderungen.

**Patentansprüche:**

1. 1.1-Dimethoxy-2.5-dimethyl-2-vinyl-4-hexen, 1.1-Dimeth-oxy-2.2.5-trimethyl-4-hexen und 1.1-Dimethoxy-2.2-di-methyl-3-phenyl-propan.

2. Verfahren zur Herstellung der Verbindungen nach Anspruch 1, dadurch gekennzeichnet, daß 2.5-Dimethyl-2-vinyl-4-hexenal, 2.2.5-Trimethyl-4-hexenal oder 2.2-Dimethyl-3-phenyl-propanal mit Methanol in Gegenwart von wasserfreiem Calziumsalz umgesetzt werden.

3. Verwendung der Verbindungen nach Anspruch 1 als Riechstoffe oder Bestandteil von Riechstoffmischungen.

**Claims**

1. 1,1-dimethoxy-2,5-dimethyl-2-vinyl-4-hexene, 1,1-dimethoxy-2,2,5-trimethyl-4-hexene and 1,1-dimethoxy-2,2-dimethyl-3-phenylpropane.

2. Process for the manufacture of the compounds according to claim 1, characterised in that 2,5-dimethyl-2-vinyl-4-hexenal, 2,2,5-trimethyl-4-hexenal or 2,2-dimethyl-3-phenylpropanal is reacted with methanol in the presence of an anhydrous calcium salt.

3. Use of the compounds according to claim 1 as perfumes or components of perfume mixtures.

**Revendications**

1 1.1-diméthoxy-2.5-diméthyl-2-vinyl-4-hexène, 1.1-diméthoxy-2.2.5-triméthyl-4-hexène et 1.1-diméthoxy-2.2-di-méthyl-3-phényl-propane.

2. Procédé de préparation des composés suivant la revendication 1, caractérisé en ce qu'on fait réagir le 2.5-diméthyl-2-vinyl-4-hexènal, 2.2.5-triméthyl-4-hexènal ou le 2.2-diméthyl-3-phényl-propanal avec du méthanol en présence d'un sel de calcium anhydre.

3. Utilisation des composés suivant la revendication 1, comme parfums ou comme constituants de mélanges de parfums.